# EUROPEAN PATENT SPECIFICATION

(11) **EP 1 869 066 B1**
(45) Date of publication and mention of the grant of the patent: **11.11.2009**
(21) Application number: 06724190.1
(22) Date of filing: 10.04.2006
(51) Int. Cl.: C07K 1/04, C07K 14/16, C07K 17/08, C07K 17/06

(54) **PEPTIDE SYNTHESIS OF ALPHA-HELIXES ON PEG RESIN**
PEPTIDSYNTHESE VON ALPHA-HELICES AUF PEG-HARZ
SYNTHESE PEPTIDIQUE D'HELICES ALPHA SUR RESINE PEG

(30) Priority: 08.04.2005 EP 05007694; 09.05.2005 EP 05010051; 18.07.2005 US 699861 P
(43) Date of publication of application: 26.12.2007
(73) Proprietor: LONZA AG, CH-4002 Basel (CH)
(72) Inventor: ALBERICIO, Fernando, E-08007 Barcelona (ES); CRUZ, Luis, Javier, E-08028 Barcelona (ES); GARCIA-MARTIN, Fayna, E-28230 Las Rozas C.P. Madrid (ES); TULLA-PUCHE, Judit, E-08028 Barcelona (ES)
(86) International application number: PCT/EP2006/003256
(87) International publication number: WO 2006/108594

(56) References cited:
- WO-A-01/76615
- WO-A-03/093786
- WO-A-2004/013164
- WO-A-2004/085505
- WO-A-2004/094465
- WO-A-2005/012277
- US-A1- 2003 078 372
- US-B1- 6 281 331
- US-B1- 6 458 764
- US-B1- 6 682 736
- FISHER L E; ENGELMAN D M: "High-yield synthesis and purification of an alpha-helical transmembrane domain" ANALYTICAL BIOCHEMISTRY, vol. 293, no. 1, 1 June 2001 (2001-06-01), pages 102-108, XP002387115
- KIYOTA T; LEE S; SUGIHARA G: "Design and synthesis of amphiphilic alpha-helical model peptides with systematically varied hydrophobic-hydrophilic balance and their interaction with lipid- and bio-membranes" BIOCHEMISTRY, vol. 35, no. 40, 8 October 1996 (1996-10-08), pages 13196-13204, XP002387116
- CHOWDHURY FARHANA A; RALEIGH DANIEL P: "A comparative study of the alpha-subdomains of bovine and human alpha-lactalbumin reveals key differences that correlate with molten globule stability" PROTEIN SCIENCE, vol. 14, no. 1, January 2005 (2005-01), pages 89-96, XP002387117
- CRUMP M P ET AL: "SOLUTION STRUCTURE AND BASIS FOR FUNCTIONAL ACTIVITY OF STROMAL CELL-DERIVED FACTOR-18: DISSOCIATION OF CXCR4 ACTIVATION FROM BINDING AND INHIBITION OF HIV-1" EMBO JOURNAL, OXFORD UNIVERSITY PRESS, SURREY, GB, vol. 16, no. 23, 1997, pages 6996-7007, XP002915918 ISSN: 0261-4189
- CLORE G M ET AL: "SOLUTION STRUCTURE OF HUMAN GROWTH HORMONE RELEASING FACTOR COMBINED USE OF CIRCULAR DICHROISM AND NUCLEAR MAGNETIC RESONANCE SPECTROSCOPY" JOURNAL OF MOLECULAR BIOLOGY, LONDON, GB, vol. 191, no. 3, 1986, pages 553-561, XP008065795 ISSN: 0022-2836

### SEQUENCE LISTING

<110> Lonza AG
   <120> Method of peptide synthesis
   <130> LP2045
   <160> 2
   <170> PatentIn version 3.3
<210> 1
   <211> 36
   <212> PRT
   <213> Artificial
<220>
   <223> Synthetic construct
<400> 1
   Tyr Thr Ser Leu Ile His Ser Leu Ile Glu Glu Ser Gln Asn Gln Gln
   1 5 10 15
   Glu Lys Asn Glu Gln Glu Leu Leu Glu Leu Asp Lys Trp Ala Ser Leu
   20 25 30
   Trp Asn Trp Phe
   35
<210> 2
   <211> 28
   <212> PRT
   <213> Artificial
<220>
   <223> synthetic construct
<400> 2
   Ser Asp Ala Ala Val Asp Thr Ser Ser Glu Ile Thr Thr Lys Asp Leu
   1 5 10 15
   Lys Glu Lys Lys Glu Val Val Glu Glu Ala Glu Asn
   20 25

## Description

The present invention relates to a method for synthesizing particular types of peptide on a solid phase, and to the respective peptide solid-phase conjugates.

A successful peptide drug, acting as an inhibitor of such a widespread human viral disease such as HIV infection, is T-20 (also named DP-178), which is Ac-Tyr-Thr-Ser-Leu-Ile-His-Ser-Leu-Ile-Glu-Glu-Ser-Gln-Asn-Gln-Gln-Glu-L s-Asn-Glu-Gln-Glu-Leu-Leu-Glu-Leu-Asp-Lys-Trp-Ala-Ser-Leu-Tip-Asn-Trp-Phe-NH₂ . The sequence of the peptide is taken from an α-helical, fusogenic sequence segment, a so-called 'heptad repeat', corresponding to amino acids 638-673 of the transmembrane receptor protein gp41 from HIV-1. Gp41 is crucial for mediating viral infection of cells.

Accordingly, efficient large scale production is needed but has so far only been achieved by a combination of partial synthesis of segments of T-20 on solid phase followed by segment condensation typically in the liquid phase (see WO 99/48513). Efficient full-length synthesis on solid-phase has not been demonstrated so far, such approach typically failing to produce more proper product than useless side products, purification of impurities posing further problems and again diminishing final yields.

Tam, J. et al. (Dep. Microbiology Vanderbilt University; Organic Letters 2002, Vol. 4; 4167-4170) describes inter-alia, the preparation of DP-178 and related 30-me peptides with the purpose of preparing three-helix bundles.
Authors just mentioned that a general FMOC synthesis method is applied, giving the coupling reagents and resin cleavage conditions used. Nothing is said on the type of resin and/or resin-handle employed, as well as the excess of reagents used. No chromatogram is shown and no purification details nor final purity is given. No indication of yields obtained are given and are entirely left subject to speculation. Since the work pertained only to lab-scale experimentation, no apparent need for devising an efficient synthetic process was given though.

It is an object of the present invention to devise an improved, efficient method for synthesis of such a-helical proteins and in particular T-20. This object is solved by the method of the present invention.

According to the method of the present invention for preparing a peptide, it comprises the step in that said peptide which may have protected and/or unprotected amino acid side chains is prepared bound to a solid phase which solid phase is an amphiphilic PEG resin and wherein the peptide comprises a prolin-free, helix-forming segment consisting of a run of six contiguous amino acid residues comprising at least four protected or unprotected helix-forming amino acid residues.

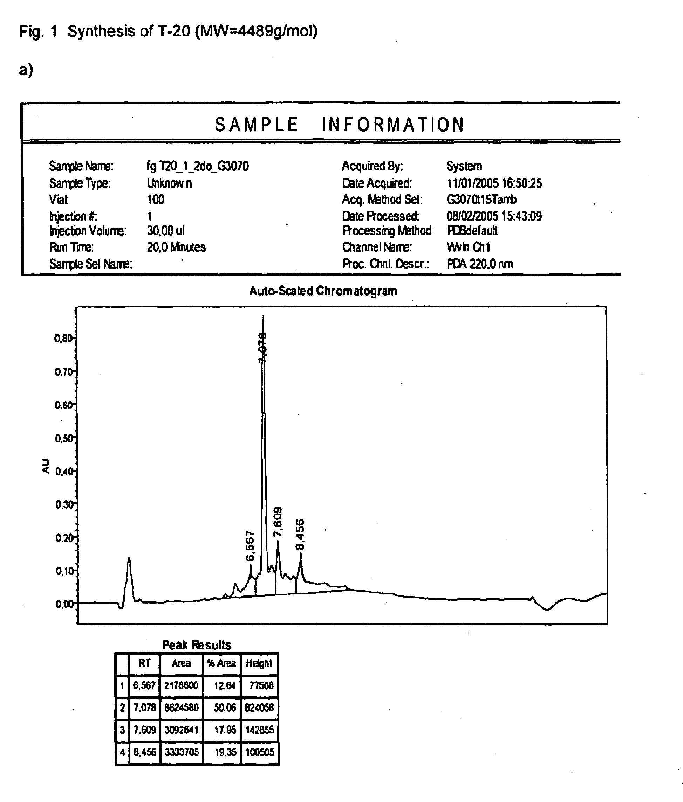

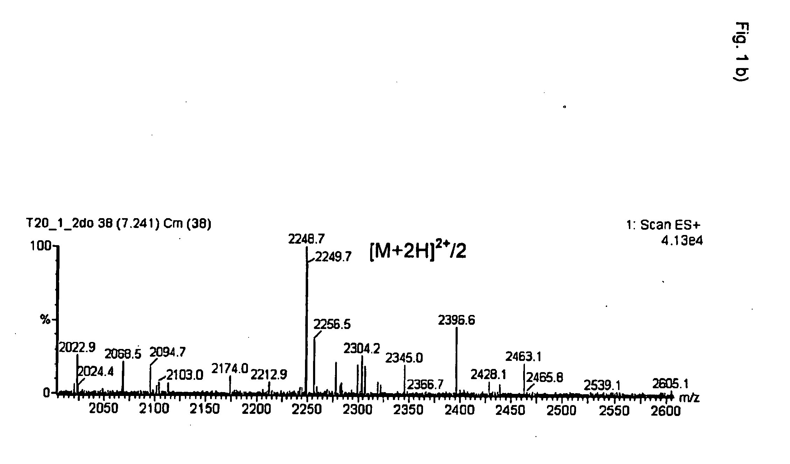

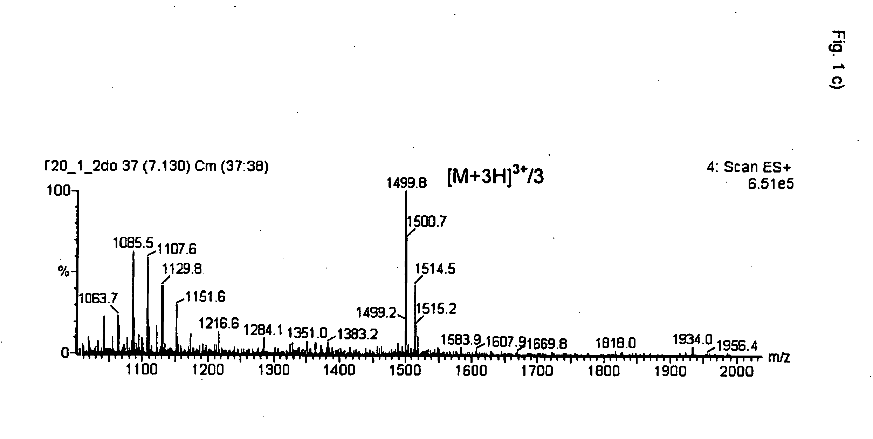

Fig. 1 shows the HPLC chromatogram and low level of impurities obtained from the product synthesized in this way, allowed of high-yield synthesis.
PEG resins are offered by different companies, e.g. Matrix Innovations Inc. from Quebec, Canada ('ChemMatrix' brand) or Versamatrix A.S. from Denmark. Preferred types of PEG resins have been described in WO 02/40559. For linkage of peptide, such PEG resin may comprise terminal 'native' hydroxymethyl radicals or derived thereof 'quasi-native' aminomethyl, carboxyl or bromomethyl or iodomethyl radicals for instance, or may be derivatized by known integral or grafted linkers or handles for solid phase linkage such as e.g. Wang resins, 4-hydroxymethylbenzoic acid (the latter requiring attachment of the first amino acid by means of p-dimethylaminopyridine catalysed esterification, see Atherton et al., 1981, J. Chem. Soc. Chem. Commun., p.336 ff), 2-chlorotritly chloride (CTC) and related, preferably alkoxylated, trityl halogenid resins, Bayer's 4-carboxytrityl linker, 4-methylbenzylhydrylamine resin, or e.g. the amide generating PAL, Rink or Sieber amide linkers as are used in Fmoc chemistry, or where Boc chemistry is to be used, e.g. Pam handle or the amide-generating BHA handle.

Preferably, the PEG resin used in the present invention comprises a linker comprising a reactive NH radical which generates upon cleavage of peptide from resin an acidamide, be it after initial coupling of the Cα-carboxylic acid function of the C-tenninal amino acid residue of the peptide to such linker or be it from the ω-carboxylic acid side chain function of aspartic or glutamic acid, yielding asparagine or glutamine upon cleavage after initial side-chain anchoring and projection of the C-terminus of the peptide. In the present context, such linkers are named amide-generating linkers. More preferably, and particularly for synthesis of T-20, such amide-generating linker is PAL (Albericio et al., 1987, Int. J. Pept. Protein Research 30, 206-216), Sieber (Tetrahedron Lett. 1987, 28, 2107-2110) or similar 9-aminoxanthenyl-type resin linker, or Rink amide (4-(2',4'-dimethoxyphenyl-Fmoc-aminomethyl)phenoxy; Rink et al, 1987, Tetrahedron Lett. 28 3787 ff) linker.

The PEG resin is a substantially pure PEG resin that is devoid of eventually further substituted polystyrene moieties as a defining structural element and, further does also not comprise any internal ester or amide functional groups but only polyether functional groups. Notably, in the latter definition terminal ester or amide bonds bridging the resin directly or via a handle to the peptide are not accounted for, since they are not internal structural, cross linking features and accordingly do not affect the definition of 'pure PEG' in this regard.

PEG resins offer optimal chemical stability along with good compatibility and ease of handling with standard solvents during normal Fmoc synthesis. According to the present inventions, they allow of synthesizing e.g. T-20 in exceptional yield and purity.

A helix-forming segment comprises helix-forming amino acid residues as defined above; helix-forming residues in their meaning in the present context are those amounting to strong helix formers, namely being annotated as Hα or ha-type amino acids in the helix propensity annotation and prediction scheme according to Chou and Fasman, Ann. Rev. Biochem. 47, 25 8 (1975). Such strong helix formers simply have an empirically determined helical propensity of >1 indicating that such strong helix forming amino acid residue occurs with greater than average frequency in an α helix in X-ray protein structures. For each of the natural 20 amino acids, an empirical numerical propensity value can be found tabulated in Chou, supra. Strong helix formers accordingly are Ala, Gln, Glu, Ile, Leu, Lys, Met, Phe, Trp and Val. In contrast, His, Arg and Asp occured with statistical frequency, meaning a propensity of about 1, in α-helices whilst Asn was found to have a truly negative impact on helix formation, namely a propensity of 0.67 only. For prediction of helical or helix-forming peptide segments, a comparatively simple but reliable prediction algorithm was defined by Chou et al, 1978: A cluster of 4 strongly helix forming amino acid residues, with the exceptional rule that two His and/or Asp make up for one strong helix forming residue, within six contiguous amino acid residues will nucleate a helix. Calculating the average helix propensity for every overlapping tetrapeptide segment of such helical segment, such helix may then be predicted to extend to both directions until the averaged propensity for the terminal tetrapeptide segment falls below 1.00. Prolines are helix-breakers and may only occur at the ends of a helix. Preferably, an accordingly predicted helix-forming segment has an average helix propensity that is larger than a similiarly predictable averaged beta-sheet propensity value according to Chou, supra.
Whilst it has often been contemplated in the field of peptide synthesis that beta-sheet structures, leading to aggregation of adjacent peptide chains by inter-chain bonding, may negatively affect coupling reactions during and deprotection reactions after synthesis, intra-chain helix formation has not been perceived as a likely obstacle for linear solid-phase synthesis.

Coupling reactions, coupling reagents employed and cleavage from resin/side chain deprotection are to be conducted with standard solid phase synthesis protocols, employing e.g. Boc or, preferably, Fmoc chemistry. Notably, coupling efficiency after each coupling step should be controlled during synthesis by means of e.g. ninhydrin test and individual couplings showing unexpected low coupling efficiency should be repeated prior to continuing with further cycle of deprotection and coupling. In particular after critical coupling steps, pre-emptive capping of still unreacted Nα-functions could be favorably carried out prior to the next synthetic cycle.

After on-resin synthesis, in particular in case of T-20; N-temrinal acetylation commonly using acetic anhydride or similar modification may be carried out. After usually one-step global cleavage from PEG resin and deprotection where not using .special, orthogonal protecting groups for side chains, the peptide is harvested and isolated or further processed by customary methods.

Preferably, the peptide to be synthesized is T-20. However, other peptides comprising helix-forming segments are also accessible by the present method. Apart from T-20, further examples of similar peptides having such helix-fonning segments are overlapping or related, but not identical peptides from said gp41 heptad repeat domains having 15-40 amino acid residues and overlapping with T-20 by at least 10 amino acid residues.

Preferably, in addition to the requirement of comprising a helical peptide segment, the total length of the peptide to be synthesized and which peptides comprises aforesaid helical peptide segment as specified is at least 15 amino acids, more preferably it is at least 24 amino acids, most preferably it is at least 30 amino acids long.

Preferably, the peptide to be synthesized is Thymosin α₁ having the sequence
1-Ser-Asp-Ala-Ala-Va1-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-28
wherein the individual amino acid chains are unprotected or are suitably protected, as need may be for a given individual amino acid residue as is common knowledge in the art (see Bodanseky, infra, for instance). More preferably, such peptide is N-terminally acetylated in a step subsequent to linear synthesis, but prior to cleavage from resin. Natural hymalfasin as is also used as a pharmaceutical is N-terminally acetylated.

Notably the present inventors have found that it is mainly the C-terminal half of the thymalfasin peptide, it is about residues 19-28, that incur most of the impurities by chain deletions and hence losses in final product yield generated during linear phase synthesis. This part of the peptide has been found to be extremely difficult to synthesize.

Vice versa, during linear synthesis, it is also feasible to use such oxazolidine dipeptides according to Wöhr et al., (Wöhr et al., J. Am. Chem. Soc. 118, 9218).

### EXAMPLES

### Solid-phase synthesis of T-20

General Procedures. ChemMatrix resin from Matrix Innovation (Quebec, Canada), HCTU from Luxembourg Industries Ltd. (Tel Aviv, Israel), protected Fmoc-amino acid derivatives and Rink handle from IRIS Biotech (Marktredwitz, Germany).
Manual solid-phase syntheses were carried out in polypropylene syringes (10 mL) fitted with a polyethylene porous disc. Solvents and soluble reagents were removed by suction. Removal of the Fmoc group was carried out with piperidine-DMF (2:8, v/v) (1 x 2 min, 2 x 10 min). Washings between deprotection, coupling, and, again, deprotection steps were carried out with DMF (5 x 0.5 min) and CH₂Cl₂ (5 x 0.5 min) using each time 10 mL) solvent/g resin. Peptide synthesis transformations and washes were performed at 25 °C.
Automatic solid-phase syntheses were carried out in an ABI 433A peptide synthesizer using a FastMoc program using 0.10 mmol of resin.
*Removal* of the Fmoc was carried out with 22% piperidine in DMF for 2 + 7.6 min. The resin was washed with DCM (6 times for 0.5 min) and (4 times).
*Coupling.* In a cartridge, Fmoc-amino acid (0.9 mmol, 10 equiv) was dissolved in DMF and 0.9 mmol of 0.45 M HCTU in DMF were added to the cartridge. Then, 1 mL of 2 M DIEA in NMP was added and the solution was transferred to the reaction vessel. The coupling take place for 30 min, where the resin was washed (6 times) with DMF.

HPLC reversed phase columns Symmetry^{™} C₁₈ 4,6 x 150 mm, 5 µm (column A) and were from Waters (Ireland). Analytical HPLC was carried out on a Waters instrument comprising two solvent delivery pumps (Waters 1525), automatic injector (Waters 717 autosampler), dual wavelength detector (Waters 2487), and system controller (Breeze V3.20). UV detection was at 220 nm, and linear gradients of CH₃CN (+0.036% TFA) into H₂O (+0.045% TFA), from 30% to 70% in 15 min.
MALDI-TOF and ES-MS analyses of peptide samples were performed in a PerSeptive Biosystems Voyager DE RP, using ACH matrix. Fig. 1 shows purity and yield of product in a) HPLC chromatogram and b,c) MS spectra. Yield of pure product (RT 7,078 was 50% relative to impurities in HPLC analysis.

### Example 1

### Fmoc-Rink-ChemMatrix-resin

ChemMatrix-resin (0.1 mmol, 0.45 mmol/g) was placed in the polypropylene syringe. The resin was subjected to the following washings/treatments with CH₂Cl₂ (3 x 0.5 min), DMF (3 x 0.5 min), and DMF (5 x 0.5 min). Then, Fmoc-Rink handle (3 equiv) and DIEA (6 equiv) in DMF (1 mL) were added, followed by PyBOP (3 equiv) and HOAt (3 equiv) in DMF (5 mL). The mixture was left to stir mechanically for 2 h and the resin was washed with DMF (3 x 0.5 min) and the ninhydrin test was negative.

The Fmoc group was removed and Fmoc-aa-OH was sequentially added to the above peptidyl-resin (step 1) in the ABI automatic synthesizer 433A as described above.

The final acetylation was carried out with a solution of Ac₂O-DIEA-DMF (10:5:85) in DMF (5 mL) for 15 min with sporadic manual stirring, where ninhydrin was negative.

The peptide-resin was suspended in TFA-iPr₃SiH-H₂O (95:2.5:2.5) and the mixture was allowed to stir for 2 h. Then, ether/hexane was added, the peptide precipitated, the solvent was removed after centrifugation, and extra ether/hexane was added and removed after centrifugation. This operation was repeated several times. Finally, H₂O (5 mL) was added and lyophilized.
EI-MS, calcd. 4489. Found: *m*/*z* [M+2H]²⁺/2 2248

## Claims

1. Method of preparing a peptide on a solid phase which peptide is at least 24 amino acid residues long and which peptide may have individually protected and/or unprotected amino acid side chains, **characterized in that** said peptide is prepared bound to a solid phase which solid phase is an amphiphilic PEG-resin comprising only polyether functional groups as an internal crosslinking, structural element and wherein the peptide comprises a prolin-free, helix-forming segment which is at least 12 residues long.

2. Method according to claim 1, **characterized in that** the loading capacity of the PEG resin is at least 0.5 mmol/g.

3. Method according to claim 1, **characterized in that** the total length of the peptide to be synthesized and which peptide comprises aforesaid helical peptide segments is at least 30 amino acids.

4. Method according to claim 1, **characterized in that** the peptide is T-20, N-terminally acetylated T-20, Thymosin-αl or is a heptad repeat peptide of 15-40 amino acid residues having an α-helical, fusogenic sequence element which is overlapping with T-20 by at least 10 residues.

5. Method according to one of the preceding claims, **characterized in that** the peptide is linked to the solid phase via a functional linker or handle.

6. Method according to claim 5, **characterized in that** the linker is an amide-generating linker, preferably is a Sieber or Rink amide linker.

7. Method According to claim 1, **characterized in that** the PEG resin is an aminoalkylfunctionalized PEG resin to which amino function the peptide is bound forming a Cα-terminal peptidyl-carboxamide.

8. Method according to claim 2 or 5, **characterized in that** the resin is a PEG resin wherein the resin is not a polystyrene-PEG mixed or co-polyrner or block copolymer, preferably that the resin is a substantially pure PEG resin.

9. Conjugate of a peptide of at least 24 amino acid residues and a substantially pure PEG-resin solid phase which resin is an amphiphilic PEG-msin comprising only polyether functional groups as an internal crosslinking, structural element and wherein the peptide comprises a prolin-free, helix-forming segment which is at least 12 residues long.

10. Peptide-PEG resin conjugate according to claim 9, **characterized in that** the peptide moiety has the amino acid sequence is R1 Tyr-Thr-Ser-Leu-Ile-His-Ser-Leu-Ile-Glu-Glu-Ser-Gln-Asn-Oln-Gln-Glu-Lys-Asn-Gln-Glu-Glu-Leu-Leu-Glu-Leu-Asp-Lys-Trp-Ala=Ser-Leu-Tip=Asn-Trp-Phe and wherein R1 is H, acetyl- or a removable protecting group, most preferably R1 is acetyl, and wherein the individual amino acid residues may further comprise a side-chain protecting group.

11. Peptide-PEG resin conjugate according to claim 9, **characterized in that** the peptide moiety is Thymosin-α1.

## Patentansprüche

1. Verfahren zur Herstellung eines Peptids an einer Festphase, wobei das Peptid mindestens 24 Aminosäurereste lang ist und wobei das Peptid individuell geschützte und/oder ungeschützte Aminosäureseitenketten aufweisen kann, **dadurch gekennzeichnet, dass** das besagte Peptid an eine Festphase gebunden hergestellt wird, wobei es sich bei der Festphase um ein amphiphiles PEG-Harz umfassend ausschließlich Polyether-Funktionsgruppen als internes vernetzendes Strukturelement handelt und wobei das Peptid einen Prolin-freien, helixbildenden Abschnitt umfaßt, der mindestens 12 Reste lang ist.

2. Verfahren nach Anspruch 1, **dadurch gekennzeichnet, dass** die Beladungsfähigkeit des PEG-Harzes mindestens 0,5 mmol/g beträgt.

3. Verfahren nach Anspruch 1, **dadurch gekennzeichnet, dass** die Gesamtlänge des zu synthetisierenden Peptids, wobei das Peptid die oben genannten Helixpeptidabschnitte umfaßt, mindestens 30 Aminosäuren beträgt.

4. Verfahren nach Anspruch 1, **dadurch gekennzeichnet, dass** es sich bei dem Peptid um T-20, N-terminal acetyliertes T-20, Thymosin-α1 oder ein *"heptad repeat"Peptid* mit 15-40 Aminosäureresten mit einem α-helixförmigen fusogenen Sequenzelement handelt, das mit T-20 um mindestens 10 Reste überlappt.

5. Verfahren nach einem der vorhergehenden Ansprüche, **dadurch gekennzeichnet, dass** das Peptid an die Festphase über einen funktionellen Linker oder Griff verbunden ist.

6. Verfahren nach Anspruch 5, **dadurch gekennzeichnet, dass** es sich bei dem Linker um einen Amid-bildenden Linker, vorzugsweise um einen Sieber- oder Rink-Amidlinker handelt.

7. Verfahren nach Anspruch 1, **dadurch gekennzeichnet, dass** es sich bei dem PEG-Harz um ein Aminoalkyl-funktionalisiertes PEG-Harz handelt, an dessen Aminofunktion das Peptid unter Bildung eines Cα-terminalen Peptidylcarboxamids gebunden ist.

8. Verfahren nach Anspruch 2 oder 5, **dadurch gekennzeichnet, dass** es sich bei dem Harz um ein PEG-Harz handelt, wobei es sich bei dem Harz nicht um ein Polystyrol-PEG-Mischpolymer oder -Copolymer oder -Blockcopolymer handelt, vorzugsweise wobei es sich bei dem Harz um im wesentlichen reines PEG-Harz handelt.

9. Konjugat aus einem Peptid mit mindestens 24 Aminosäureresten und einer im wesentlichen reinen PEG-Harzfestphase, wobei es sich bei dem Harz um ein amphiphiles PEG-Harz umfassend ausschließlich Polyether-Funktionsgruppen als internes vernetzendes Strukturelement handelt und wobei das Peptid einen Prolin-freien, helixbildenden Abschnitt umfaßt, der mindestens 12 Reste lang ist.

10. Peptid-PEG-Harz-Konjugat nach Anspruch 9, **dadurch gekennzeichnet, dass** die Peptideinheit, die die Aminosäuresequenz hat, R1-Tyr-Thr-Ser-Leu-lle-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-Ser-Leu-Trp-Asn-Trp-Phe ist und wobei R1 H, Acetylgruppe oder eine entfernbare Schutzgruppe bedeutet und wobei R1 am stärksten bevorzugt Acetyl bedeutet und wobei die einzelnen Aminosäurereste ausserdem eine Seitenkettenschutzgruppe umfassen können.

11. Peptid-PEG-Harz-Konjugat nach Anspruch 9, **dadurch gekennzeichnet, dass** es sich bei der Peptideinheit um Thymosin-α1 handelt.

## Revendications

1. Procédé de préparation d'un peptide sur une phase solide, lequel peptide est long d'au moins 24 résidus d'acides aminés et lequel peptide peut avoir des chaînes latérales d'acides aminés individuellement protégés et/ou non protégés, **caractérisé en ce que** ledit peptide est préparé lié à une phase solide, laquelle phase solide est une résine PEG amphiphile constituée seulement de groupes polyéther fonctionnels comme une réticulation interne, un élément structurel, et dans lequel le peptide est constitué d'un segment en forme d'hélice sans proline qui est long d'au moins douze résidus.

2. Procédé selon la revendication 1, **caractérisé en ce que** la capacité de charge de la résine PEG est d'au moins 0,5 mmole/g.

3. Procédé selon la revendication 1, **caractérisé en ce** la longueur totale du peptide à synthétiser, lequel peptide comprend lesdits segments de peptides hélicoïdaux mentionnés ci-dessus, est d'au moins 30 acides aminés.

4. Procédé selon la revendication 1, **caractérisé en ce que** le peptide est du T-20, du T-20 acétylé en position N-terminale, de la thymosine-a1 ou est un peptide à sept motifs répétés de 15 à 40 résidus d'acides aminés qui comportent un élément de séquence fusogène α-hélicoïdale qui chevauche T-20 pendant au moins dix résidus.

5. Procédé selon l'une des revendications précédentes, **caractérisé en ce que** le peptide est lié à la phase solide par l'intermédiaire d'un lieur ou d'un motif poignée fonctionnel.

6. Procédé selon la revendication 5, **caractérisé en ce que** le lieur est un lieur générateur d'amides et qu'il s'agit de préférence d'un lieur amide Sieber ou Rink.

7. Procédé selon la revendication 1, **caractérisé en ce que** la résine PEG est une résine PEG à fonction aminoalkyle à laquelle fonction amino le peptide est lié pour former un peptidyl-carboxamide en position Ca-terminale.

8. Procédé selon les revendications 2 ou 5, **caractérisé en ce que** la résine est une résine PEG dans laquelle cette résine n'est pas un mélange polystyrène-PEG ou un copolymère ou un copolymère séquencé, de préférence la résine étant essentiellement une résine PEG pure.

9. Conjugué d'un peptide d'au moins 24 résidus d'acides aminés et une phase solide de résine PEG essentiellement pure, laquelle résine est une résine PEG amphiphile qui comprend seulement des groupes polyéther fonctionnels comme réticulation interne, un élément structural, et dans lequel le peptide comprend un segment en forme d'hélice exempt de proline qui est long d'au moins douze résidus.

10. Conjugué de peptide-résine PEG selon la revendication 9, **caractérisé en ce que** le fragment peptide possède la séquence d'acides aminés qui est R1-Tyr-Thr-Ser-Leu-Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-Ser-Leu-Trp-Asn-Trp-Phe et dans laquelle R1 représente un atome H, un groupe de protection acétyle ou amovible, idéalement R1 représentant un groupe acétyle, et dans lequel les résidus individuels d'acides aminés peuvent en outre comprendre un groupe de protection de chaîne latérale.

11. Conjugué peptide-résine PEG selon la revendication 9, **caractérisé en ce que** le fragment peptide est de la thymosine-α1.
